# EUROPÄISCHE PATENTSCHRIFT

(11) **EP 0 524 963 B1**
(45) Veröffentlichungstag und Bekanntmachung des Hinweises auf die Patenterteilung: **15.12.1993**
(21) Anmeldenummer: 91906396.6
(22) Anmeldetag: 20.03.1991
(51) Int. Cl.: A61K 9/14, A61K 9/18, A61K 9/70, A61K 47/10, A61K 47/24, A61K 47/36, A61K 47/00

(54) **VERFAHREN ZUR HERSTELLUNG FESTER WIRKSTOFF-FORMEN UND DIE SO ERHALTENEN PRODUKTE**
PROCESS FOR PRODUCING SOLID ACTIVE SUBSTANCE FORMS AND PRODUCTS SO OBTAINED
PROCEDE DE PRODUCTION DE FORMES SOLIDES EN SUBSTANCES ACTIVES ET LES PRODUITS AINSI OBTENUS

(30) Priorität: 29.03.1990 DE 4009984
(43) Veröffentlichungstag der Anmeldung: 03.02.1993
(73) Patentinhaber: KNOLL AG, D-67061 Ludwigshafen (DE)
(72) Erfinder: ROSENBERG, Joerg, D-6701 Ellerstadt (DE); HEBERGER, Juergen, D-6707 Schifferstadt (DE)
(74) Vertreter: Karau, Wolfgang, Dr.
(86) Internationale Anmeldenummer: EP9100538
(87) Internationale Veröffentlichungsnummer: WO9114425

(56) Entgegenhaltungen:
- EP-A- 0 274 431
- DE-A- 3 400 106

## Beschreibung

Die vorliegende Erfindung betrifft ein Verfahren zur Herstellung fester Wirkstoff-Formen, die mit Hilfe dieses Verfahrens erhaltenen Produkte und die Verwendung dieser Produkte zur Herstellung von Arzneimitteln.

Es ist bekannt, daß beim Mischen einer wäßrigen Lösung aus Polyethylenglykol und einer ebenfalls wäßrigen Lösung von Dextran Entmischung auftritt. Gemäß US-PS 4.794.000 kann die untere "kolloidreiche" Phase zur Formulierung von Wirkstoffen eingesetzt werden, gegebenenfalls nach Zusatz weiterer Hilfsstoffe, wie Lecithin. Alle dort beschriebenen Formulierungen sind allerdings flüssige Arzneiformen, oft hochviskos, und daher nachteilig, insbesondere für orale Applikationsformen. Zudem muß insbesondere im flüssigen Zustand mit erheblichen Stabilitätsproblemen physikalischer (Entmischung, Rekristallisation etc.) und chemischer Art (Hydrolysereaktionen, Oxidationen etc.) gerechnet werden.

Alle zur Formulierung eingesetzten Hilfsstoffe (Polyethylenglykol 6000, Dextran 40 sowie Eierlecithin) zeigen wachsartige, klebrige Eigenschaften und bereiten daher Probleme bei der Formulierung von Wirkstoffen. Wirkstoffe ihrerseits können eine Konsistenz haben, die die galenische Bearbeitung erschwert.

Es wurde nun ein sehr einfaches und gutes Verfahren zur Herstellung fester Wirkstoff-Formen gefunden.

Gegenstand der Erfindung ist ein Verfahren zur Herstellung fester Wirkstoff-Formen mit guter Redispergierbarkeit in wäßrigen Systemen, welches dadurch gekennzeichnet ist, daß man PEG und Dextran mit Wasser mischt und - falls eine Phasentrennung auftritt - die obere Phase entfernt, einen Wirkstoff in der Mischung löst, gegebenenfalls ein physiologisch verträgliches Amphiphil zusetzt und anschließend das Wasser entfernt.

Als Wirkstoffe eignen sich vor allem solche, die in Wasser löslich sind. Als PEG (= Polyethylenglykol) kommen insbesondere solche mit einem Molekulargewicht von 2.000 bis 20.000, z.B. PEG 4.000 und PEG 10.000 in Betracht. Ganz besonders eignet sich PEG 6.000.

Es empfiehlt sich, der Mischung nach Zugabe des Wirkstoffs ein Amphiphil zuzusetzen. Als Amphiphil eignen sich alle, die physiologisch verträglich sind, wie Doppelschichtmembran-bildende Lipide. Solche sind beispielsweise Phospholipide oder synthetische Amphiphile. Beispiele für synthetische Amphiphile sind in der EP-OS 331.092 beschrieben. Besonders gut eignen sich Phospholipide, vorzugsweise Lecithine wie Eier-Lecithin, Soja-Lecithin und synthetische Phospholipide.

Die Entfernung des Wassers aus dem Substanzgemisch geschieht beispielsweise durch Sprühtrocknung oder Gefriertrocknung.

Gegenstand der Erfindung ist auch die nach dem beschriebenen Verfahren erhaltene Wirkstoff-Form, die in bekannter Weise zu einer fertigen Arzneiform verarbeitet werden kann. Die Wirkstoff-Form eignet sich gut zur Herstellung von Instant-Formen, beispielsweise für Injektionszubereitungen sowie für Trinklösungen, weiter insbesondere aber auch für feste perorale Arzneiformen (nach Abfüllen des Pulvers in Hartgelatinesteckkapseln oder nach Verpressen zu Tabletten).

Es war überraschend, daß definierte Mischungen aus diesen klebrigen, wachsartigen Hilfsstoffen, auch in Kombination mit verschiedenen Wirkstoffen nach Entzug des Wassers eine harte, spröde und gut pulverisierbare Masse ergeben, die gut rieselfähig ist und daher problemlos, z.B. in Hartgelatine-Steckkapseln abgefüllt werden kann. Diese Produkteigenschaften waren wegen der Konsistenz der Ausgangsstoffe nicht vorhersehbar. überraschend war ferner, daß beim Entzug des Wassers transparente Filme gebildet werden, wenn die Lösung z.B. in einer flachen Schale getrocknet wird. Die Filme haften extrem schlecht auf Glas und lösen sich ohne weitere Behandlung selbst von der Unterlage. Auch diese Eigenschaft war wegen der Konsistenz der Ausgangsstoffe nicht vorhersehbar. Die Transparenz der wirkstoffhaltigen Filme legt die Vermutung nahe, daß der Wird off in molekulardisperser Form, quasi in Form einer "festen Lösung" vorliegt.

Die nach der vorliegenden Erfindung hergestellten festen Wirkstoff-Formen lassen sich überraschend leicht in Wasser wieder redispergieren.

Die folgenden Beispiele veranschaulichen die Erfindung:

### Beispiel 1

### a) Herstellung der kolloidreichen Unterphase

50,0 g Dextran 40 und 15,0 g Polyethylenglykol 6000 wurden mit 200 ml destilliertem Wasser versetzt. Es wurde so lange bei 80°C gerührt, bis beide Substanzen vollständig gelöst waren. Nach dem überführen in einen Scheidetrichter ließ man auf Raumtemperatur abkühlen und trennte dann die wasserklare untere Phase ab (ca. 160 ml), die obere Phase wurde verworfen.

### b) Formulierung mit Lecithin und Wirkstoff

20,0 ml der in Beispiel 1 erhaltenen Lösung wurden bei 65°C mit 0,5 g Verapamil-Hydrochlorid versetzt. Nachdem der Wirkstoff gelöst war, wurden 1,0 g Lecithin E 100 (Eierlecithin, Fa. Lipoid KG, Ludwigshafen) unter Rühren ebenfalls bei 65°C gelöst, bis eine klare, honigartige Masse gebildet war.

### c) Trocknung der Formulierung

Aus der in Beispiel 2 erhaltenen Formulierung wurde in einem Rundkolben am Rotationsverdampfer (60°C Badtemperatur, Wasserstrahlvakuum) das Wasser weitgehend entfernt. Der Kolbeninhalt wurde anschließend zur restlosen Entfernung des Wassers in einem Exsikkator über Trockenmittel (P₄O₁₀) im Vakuum 24 h nachgetrocknet. Die zurückbleibende Masse wurde schließlich in einer Reibschale pulverisiert.
- Ausbeute:: 6,84 g

### Beispiel 2

Die Präparation erfolgte wie in Beispiel 1 angegeben, aber mit 1,0 g Verapamil-Hydrochlorid.
- Ausbeute:: 7,5 g

### Beispiel 3

Die Präparation erfolgte wie in Beispiel 1 angegeben, aber mit 0,5 g Anipamil-Hydrochlorid.
- Ausbeute:: 6,75 g

### Beispiel 4

Die Präparation erfolgte wie in Beispiel 1 angegeben, aber mit 1,0 g Anipamil-Hydrochlorid.
- Ausbeute:: 7,08 g

### Beispiel 5

Die Präparation erfolgte wie in Beispiel 1 angegeben, aber mit 0,5 g Gallopamil-Hydrochlorid.
- Ausbeute:: 7,01 g

### Beispiel 6

Die Präparation erfolgte wie in Beispiel 1 angegeben, aber mit 1,0 g Gallopamil-Hydrochlorid.
- Ausbeute:: 7,33 g

### Beispiel 7

Die Präparation erfolgte wie in Beispiel 1 angegeben, aber mit 0,5 g (S)-Emopamil-Hydrochlorid.
- Ausbeute:: 7,3 g

### Beispiel 8

Die Präparation erfolgte wie in Beispiel 1 angegeben, aber mit 1,0 g (S)-Emopamil-Hydrochlorid.
- Ausbeute:: 7,6 g

### Beispiel 9

Die Präparation erfolgte wie in Beispiel 1 angegeben, aber mit 2,0 g (S)-Emopamil-Hydrochlorid.
- Ausbeute:: 8,4 g.

## Patentansprüche

1. Verfahren zur Herstellung fester Wirkstoff-Formen mit guter Redispergierbarkeit in wäßrigen Systemen, dadurch gekennzeichnet, daß man PEG und Dextran mit Wasser mischt und - falls eine Phasentrennung auftritt - die obere Phase entfernt, einen Wirkstoff in der Mischung löst, gegebenenfalls ein physiologisch verträgliches Amphiphil zusetzt und anschließend das Wasser entfernt.

2. Feste Wirkstoff-Form, erhältlich gemäß Anspruch 1.

3. Verwendung der Wirkstoff-Form gemäß Anspruch 2 zur Herstellung von Arzneimitteln.

## Claims

1. A process for producing solid drug forms with good redispersibility in aqueous systems, which comprises mixing PEG and dextran with water and, -if phase separation occurs, removing the upper phase, dissolving a drug in the mixture, where appropriate adding a physiologically tolerated amphiphile and subsequently removing the water.

2. A solid drug form obtainable as claimed in claim 1.

3. The use of the drug form as claimed in claim 2 for producing pharmaceutical compositions.

## Revendications

1. Procédé de préparation de formes de principes actifs solides à bonne redispersibilité dans des systèmes aqueux, caractérisé en ce que l'on mélange du polyéthyléneglycol et du dextrane avec de l'eau et - au cas où une séparation de phases viendrait à se produire - on enlève la phase supérieure, on dissout un principe actif dans le mélange, on ajoute éventuellement une substance amphiphile physiologiquement compatible et on élimine ensuite l'eau.

2. Forme de principe actif que l'on peut obtenir par mise en oeuvre du procédé suivant la revendication 1.

3. Utilisation de la forme de principe actif suivant la revendication 2 pour la fabrication de médicaments.
